# EUROPEAN PATENT SPECIFICATION

(11) **EP 1 849 644 B1**
(45) Date of publication and mention of the grant of the patent: **30.09.2009**
(21) Application number: 06113142.1
(22) Date of filing: 26.04.2006
(51) Int. Cl.: B60K 28/06, B60R 25/04

(54) **System and method for selective engine start in a vehicle depending on driving ability**
System und Verfahren zum wahlweisen Starten eines Motors abhängig von der Fahrtüchtigkeit
Système et procédé pour l'activation sélective du moteur selon la capacité du conducteur

(43) Date of publication of application: 31.10.2007
(73) Proprietor: Ford Global Technologies, LLC, Dearborn, MI 48126 (US)
(72) Inventor: Soininen, Martti, 416 80, Göteborg (SE); Richardsson, Carl, 417 46, Göteborg (SE)
(74) Representative: Dahnér, Christer

(56) References cited:
- EP-A- 0 805 247
- WO-A-20/05118326
- DE-A1- 19 900 032
- US-A- 4 613 845
- US-A- 6 148 094
- US-A1- 2002 062 185
- US-A1- 2003 036 823
- US-B1- 6 886 653

## Description

### TECHNICAL FIELD

The present invention is related to a system for selectively allowing engine start in a vehicle in accordance with the preamble of claim 1 see DE 19900032 A. Furthermore, the present invention relates to a method for selectively allowing engine start in a vehicle in accordance with the preamble of claim 10.

### BACKGROUND OF THE INVENTION

Detecting drug-impaired drivers has gained a high level of importance during the last decade. In most cases, the reason to identify drug-impaired individuals participating in public traffic is motivated by safety issues and legal implications caused by these individuals operating cars, machinery or other equipment. Safety and legal issues are both very important in the context of a mobile society heavily relying on motorized vehicles for transportation. Financial implications for individuals or groups of the society resulting from accidents are considerable.

One previous attempt to address this problem is provided through the currently used so called Alco locks. These are designed for persons having a court order to use an Alco lock in order to be allowed to drive a car. The known Alco locks tend to be unreliable and usually requires the driver to blow in a mouthpiece in order to be able to start the car. This setup is of course not very practical and not at all suitable for wide use. Furthermore, the currently available Alco locks do not detect other drugs or impairness due to fatigue or similar.

US 6 229 908 describes a method and an ignition interlock for preventing operation of equipment when an operator's blood-alcohol content is above a threshold value. The interlock has a blood-alcohol detector that measures intensities of wavelengths of light emerging from a finger. A microprocessor correlates these intensities with the finger's blood-alcohol content, determines whether this content is above a threshold level, and prevents the equipment from operating unless the blood-alcohol content is below the threshold. A "start" button is provided to turn on the system and initiate a blood-alcohol test in order to start equipment in which the interlock is installed. When an operator wishes to start the equipment, he or she presses the "start" button energizing the interlock system. When the energized system is ready to begin testing, a "test" light illuminates. At this point, the operator inserts his or her finger through a hole of the detection unit. Once the system is energized, a microprocessor activates the blood-alcohol detector. The microprocessor determines whether the measured blood-alcohol level is above or below the pre-programmed threshold value. The microprocessor will determine that the test is passed if the blood-alcohol reading is below the threshold value.

In this case, however, a driver is required to perform a sequence of steps, pressing a "start" button, waiting for the system indication of being ready for testing and inserting his or her finger through a hole of a detection unit.

### SUMMARY OF THE INVENTION

An object of the present invention is to provide an improved system for selectively allowing engine start in a vehicle, the system comprising a start button arranged to be touched by a driver of the vehicle for starting an engine thereof.

According to a first aspect of the present invention this object is achieved in accordance with the characterizing portion of claim 1, which specifies that it further comprises: sensing means, arranged at the start button in an area which the driver will need to touch for starting, for sensing of substance related parameters associated with a body part of the driver employed for the touching action; determination means, for determining from any sensed substance related parameters associated with the body part the presence and concentration of one or more specific substances likely to negatively affect the drivers suitability to drive; selective starting means, for either preventing starting of the vehicle engine, should it be determined that the presence of the one or more substances has a concentration that exceeds a predetermined threshold value, or otherwise allowing starting of the vehicle engine.

A further object of the present invention is to provide an improved method for selectively allowing engine start in a vehicle comprising a start button arranged to be touched by a driver of the vehicle for starting an engine thereof.

According to a second aspect of the present invention this object is achieved in accordance with the characterizing portion of claim 10, which specifies that the method comprises the steps of: arranging sensing means at the start button in an area which the driver will need to touch for starting, for sensing of substance related parameters associated with a body part of the driver employed for the touching action; providing determination means, for determining from any sensed substance related parameters associated with the body part the presence and concentration of one or more specific substances likely to negatively affect the drivers suitability to drive; providing selective starting means, for either preventing starting of the vehicle engine, should it be determined that any presence of the one or more substances has a concentration that exceeds a predetermined threshold value, or otherwise allowing starting of the vehicle engine.

Further embodiments are listed in the dependent claims.

It will be appreciated that features of the invention are susceptible to being combined in any combination without departing from the scope of the invention as defined by the accompany claims.

### BRIEF DESCRIPTION OF THE DRAWINGS

By way of example only, embodiments of the present invention will now be described with reference to the accompanying drawings wherein:

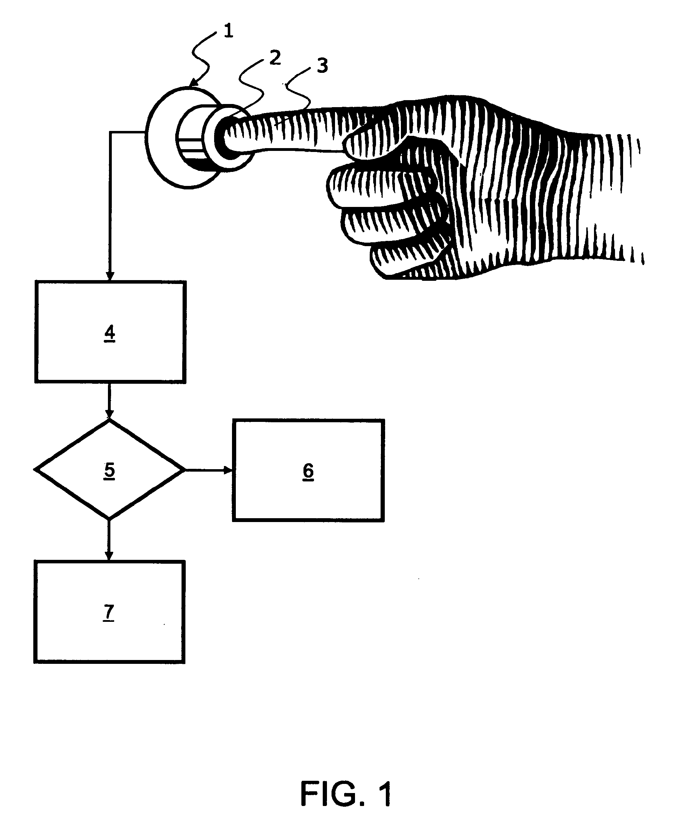

Figure 1 is a schematic illustration of a preferred embodiment of the system for selectively allowing engine start in a vehicle in accordance with present invention.

Still other objects and features of the present invention will become apparent from the following detailed description considered in conjunction with the accompanying drawings. It is to be understood, however, that the drawings are designed solely for purposes of illustration and not as a definition of the limits of the invention, for which reference should be made to the appended claims. It should be further understood that the drawings are not necessarily drawn to scale and that, unless otherwise indicated, they are merely intended to conceptually illustrate the structures and procedures described herein. The same reference numerals will be used for illustrating corresponding features in the different drawings.

### DETAILED DESCRIPTION OF PREFERRED EMBODIMENTS

In a preferred first embodiment of the present invention, as shown schematically in FIG. 1, is shown a system for selectively allowing engine start in a vehicle, such as a an automobile, a truck or any other vehicle having an engine, e.g. an internal combustion engine, an electrical engine, a hybrid engine system or similar engine system for the propulsion thereof. The system will prevent a human being from operating a motor vehicle if that human being is intoxicated or under the effects of hallucinating drugs or otherwise impaired.

The system comprises a start button 1, which is conveniently arranged at the dashboard or other suitable location where it is easily accessible by a driver of the vehicle. The start button 1 is arranged to be touched by a driver of the vehicle for starting an engine of the vehicle for allowing the vehicle to be operated.

Sensing means 2 are arranged at the start button 1 in an area which the driver will need to touch for starting the vehicle, e.g. a central area of the starting button 1. These sensing means 2 are arranged for sensing of substance related parameters associated with a body part 3 of the driver employed for the touching action, usually, as shown, an index finger or similar. The sensing means 2 may e.g. perform trans-dermal or epidermal measurements from the body part 3 of the driver employed for the touching action. The sensing means 2 may comprise a galvanic skin sensor, a spectroscopic detector sensor, an optical skin sensor or any other suitable sensor.

The sensing means 2 may be arranged for sensing of alcohol related parameters, e.g. arranged to determine from any sensed alcohol related parameters of the body part 3 of the driver employed for the touching action a blood-alcohol content of the driver, and to compare the determined blood-alcohol content of the driver with the predetermined threshold value.

The sensing means 2 may further be arranged for sensing drug related parameters, e.g. arranged for sensing any drug related parameters based on the detection of one or more chemicals present at the body part 3 of the driver employed for the touching action. The system may further be arranged to determine from any sensed drug related parameters of the body part 3 of the driver employed for the touching action a blood-chemical content of the driver, and to compare the determined blood-chemical content of the driver with the predetermined threshold value.

The sensing means 2 may further be arranged for sensing drowsiness related parameters, e.g. arranged for sensing any drowsiness related parameters based on the detection of one or more hormones, such as melatonin, present at the body part 3 of the driver employed for the touching action, e.g. a blood-melatonin content of the driver. It is known that the blood-melatonin content is instrumental in the human sleep control system, and that a high content of melatonin usually indicates that the person is sleeping or at least sleepy.

There may also be provided a temperature measurement by the sensing means 2, so that a driver cannot defeat the system by putting on special gloves to avoid detection of substance related parameters because the body part 3 of the driver employed for the touching action must be of human temperature in order for the system to work. Alternatively a fingerprint reader may be integrated into the sensing means 2 for serving the same purpose.

The sensing means 2 or sensors commonly used for these and similar purposes today may require that the body part 3 of the driver employed for the touching action is retained in contact with the area which the driver will need to touch for starting the vehicle for one or more seconds in order to complete the sensing of substance related parameters. However, it is envisaged that in phase with senor development the time required for such sensing will be reduced further.

Determination means 4 are arranged for determining from any sensed substance related parameters associated with the body part 3 the presence and concentration of one or more specific substances likely to negatively affect the driver's suitability to drive. These determination means 4 preferably comprise a microprocessor which electronically controls the functioning of the system. The microprocessor may be arranged to employ algorithms that identify distinctive features of predefined substances and compare established presences' and concentrations to threshold values which are stored in a memory associated with the microprocessor. For calibration reasons the sensed substance related parameters of a particular driver may be compared to characteristics of that same driver stored in a database.

Selective starting means 5 are arranged for either preventing 6 starting of the vehicle engine, should it be determined that the presence of the one or more substances has a concentration that exceeds a predetermined threshold value, or otherwise allowing 7 starting of the vehicle engine. These selective starting means 5 may be integrated in a usage authorisation part of an electrical system of the vehicle, which regulates all permissions to start and drive the vehicle, e.g. as the today commonly utilized transponder systems for engine start. Starting of the vehicle engine may in the case of an internal combustion engine e.g. be prevented through opening an ignition circuit of the vehicle shutting off the electrical current to the vehicle spark plugs. In a diesel engine this could also be a switch that cuts off the fuel supply to the engine, shutting off the engine.

Other measures are envisaged which may be taken by the system in response to the determined presence of one or more substances having a concentration that exceeds a predetermined threshold value. Such measures include degrading the operability of the vehicle and/or providing information to the driver of the presence of any detected substances. Information to the driver on the outcome of the determination may be provided visually, e.g. as a red/green light indication, a textual message or similar, or acoustically, e.g. as a spoken message, a tone signal or similar, or even as a combination of visual and acoustic information.

In accordance with the present invention is also envisaged an automotive vehicle, which comprises a system for selectively allowing engine start in a vehicle as described above.

Furthermore, in accordance with the present invention is also envisaged a method for selectively allowing engine start in a vehicle comprising a start button 1 arranged to be touched by a driver of the vehicle for starting an engine thereof. The method comprises the steps of: arranging sensing means 2 at the start button 1 in an area which the driver will need to touch for starting, for sensing of substance related parameters associated with a body part 3 of the driver employed for the touching action; providing determination means 4, for determining from any sensed substance related parameters associated with the body part 3 the presence and concentration of one or more specific substances likely to negatively affect the drivers suitability to drive; providing selective starting means 5, for either preventing 6 starting of the vehicle engine, should it be determined that any presence of the one or more substances has a concentration that exceeds a predetermined threshold value, or otherwise allowing 7 starting of the vehicle engine.

Modifications to embodiments of the invention described in the foregoing are possible without departing from the scope of the invention as defined by the accompanying claims.

Expressions such as "including", "comprising", "incorporating", "consisting of", "have", "is" used to describe and claim the present invention are intended to be construed in a non-exclusive manner, namely allowing for items, components or elements not explicitly described also to be present. Reference to the singular is also to be construed to relate to the plural and vice versa.

Numerals included within parentheses in the accompanying claims are intended to assist understanding of the claims and should not be construed in any way to limit subject matter claimed by these claims.

Thus, while there have been shown and described and pointed out fundamental novel features of the invention as applied to a preferred embodiment thereof, it will be understood that various omissions and substitutions and changes in the form and details of the devices illustrated, and in their operation, may be made by those skilled in the art. For example, it is expressly intended that all combinations of those elements and/or method steps which perform substantially the same function in substantially the same way to achieve the same results are within the scope of the invention. Moreover, it should be recognized that structures and/or elements and/or method steps shown and/or described in connection with any disclosed form or embodiment of the invention may be incorporated in any other disclosed or described or suggested form or embodiment as a general matter of design choice. It is the intention, therefore, to be limited only as indicated by the scope of the claims appended hereto.

## Claims

1. A system for selectively allowing engine start in a vehicle, the system comprising a start button (1) arranged to be touched by a driver of the vehicle for starting an engine thereof
**characterised in that** it further comprises:
sensing means (2), arranged at the start button (1) in an area which the driver will need to touch for starting, for sensing of substance related parameters associated with a body part (3) of the driver employed for the touching action;
determination means (4), for determining from any sensed substance related parameters associated with the body part (3) the presence and concentration of one or more specific substances likely to negatively affect the drivers suitability to drive;
selective starting means (5), for either preventing (6) starting of the vehicle engine, should it be determined that the presence of the one or more substances has a concentration that exceeds a predetermined threshold value, or otherwise allowing (7) starting of the vehicle engine.

2. A system according to claim 1, **characterised in that:**
the system is arranged for sensing of alcohol related parameters.

3. A system according to claim 2, **characterised in that**:
the system is arranged to determine from any sensed alcohol related parameters of the body part (3) of the driver employed for the touching action a blood-alcohol content of the driver, and to compare the determined blood-alcohol content of the driver with the predetermined threshold value.

4. A system according to claim 1 **characterised in that:**
the system is arranged for sensing drug related parameters.

5. A system according to claim 4, **characterised in that**:
the system is arranged for sensing any drug related parameters based on the detection of one or more chemicals present at the body part (3) of the driver employed for the touching action.

6. A system according to claim 5, **characterised in that**:
the system is arranged to determine from any sensed drug related parameters of the body part (3) of the driver employed for the touching action a blood-chemical content of the driver, and to compare the determined blood-chemical content of the driver with the predetermined threshold value.

7. A system according to any one of claims 1 to 6, **characterised in that:**
the system is arranged for sensing drowsiness related parameters.

8. A system according to claim 7, **characterised in that**:
the system is arranged for sensing any drowsiness related parameters based on the detection of one or more hormones present at the body part (3) of the driver employed for the touching action.

9. An automotive vehicle **characterised in that:**
it comprises a system according to any one of the preceding claims.

10. A method for selectively allowing engine start in a vehicle comprising a start button (1) arranged to be touched by a driver of the vehicle for starting an engine thereof, **characterised in that** it comprises the steps of:
arranging sensing means (2) at the start button (1) in an area which the driver will need to touch for starting, for sensing of substance related parameters associated with a body part (3) of the driver employed for the touching action;
providing determination means (4), for determining from any sensed substance related parameters associated with the body part (3) the presence and
concentration of one or more specific substances likely to negatively affect the drivers suitability to drive;
providing selective starting means (5), for either preventing (6) starting of the vehicle engine, should it be determined that any presence of the one or more substances has a concentration that exceeds a predetermined threshold value, or otherwise allowing (7) starting of the vehicle engine.

## Patentansprüche

1. System zum wahlweisen Zulassen des Motorstarts in einem Fahrzeug, umfassend einen Startknopf (1), der so angeordnet ist, dass er von einem Fahrer des Fahrzeugs berührt wird, um dessen Motor anzulassen, **dadurch gekennzeichnet, dass** es weiter umfasst:
Sensormittel (2), die an dem Startknopf (1) in einem Bereich angeordnet sind, den der Fahrer zum Anlassen berühren muss, zum Erkennen von substanzbezogenen Parametern, die einem Körperteil (3) des Fahrers, das für den Berührvorgang benutzt wird, zugeordnet sind,
Bestimmungsmittel (4), um aus beliebigen der erkannten, dem Körperteil (3) zugeordneten, substanzbezogenen Parameter das Vorhandensein und die Konzentration von einer oder mehrerer Substanzen zu ermitteln, welche die Eignung des Fahrers zum Fahren negativ beeinflussen können,
wählende Anlassmittel (5), die entweder das Anlassen des Motors des Fahrzeugs verhindern (6), falls ermittelt wird, dass eine oder mehrere Substanzen in einer Konzentration vorhanden ist oder sind, welche einen vorbestimmten Grenzwert übersteigt, oder andernfalls das Anlassen des Motors des Fahrzeugs zulassen (7).

2. System nach Anspruch 1, **dadurch gekennzeichnet, dass** das System auf das Erkennen von auf Alkohol bezogenen Parametern ausgelegt ist.

3. System nach Anspruch 2, **dadurch gekennzeichnet, dass** das System darauf ausgelegt ist, aus beliebigen erkannten, auf Alkohol bezogenen Parametern des für den Berührvorgang verwendeten Körperteils (3) des Fahrers einen Blutalkoholgehalt des Fahrers zu ermitteln und den ermittelten Blutalkoholgehalt des Fahrers mit dem vorbestimmten Grenzwert zu vergleichen.

4. System nach Anspruch 1, **dadurch gekennzeichnet, dass** das System auf das Erkennen von auf Drogen bezogenen Parametern ausgelegt ist.

5. System nach Anspruch 4, **dadurch gekennzeichnet, dass** das System darauf ausgelegt ist, auf Drogen bezogene Parameter aus dem Feststellen von einer oder mehreren Chemikalien zu erkennen, die an dem für den Berührvorgang verwendeten Körperteil (3) des Fahrers vorhanden ist oder sind.

6. System nach Anspruch 5, **dadurch gekennzeichnet, dass** das System darauf ausgelegt ist, aus beliebigen erkannten, auf Drogen bezogenen Parametern des für den Berührvorgang verwendeten Körperteils (3) des Fahrers einen Blut-Chemikalien-Gehalt des Fahrers zu ermitteln und den ermittelten Blut-Chemikalien-Gehalt des Fahrers mit dem vorbestimmten Grenzwert zu vergleichen.

7. System nach einem der Ansprüche 1 bis 6, **dadurch gekennzeichnet, dass** das System auf das Erkennen von auf Schläfrigkeit bezogenen Parametern ausgelegt ist.

8. System nach Anspruch 7, **dadurch gekennzeichnet, dass** das System darauf ausgelegt ist, auf Schläfrigkeit bezogene Parameter aus dem Feststellen von einem oder mehreren Hormonen zu erkennen, das bzw. die an dem für den Berührvorgang verwendeten Körperteil (3) des Fahrers vorhanden ist oder sind.

9. Automobil, **dadurch gekennzeichnet, dass** es ein System nach einem der vorhergehenden Ansprüche umfasst.

10. Verfahren zum wahlweisen Zulassen des Motorstarts in einem Fahrzeug, das einen Startknopf (1) umfasst, der so angeordnet ist, dass er von einem Fahrer des Fahrzeugs berührt wird, um dessen Motor anzulassen, **dadurch gekennzeichnet, dass** es folgende Schritte umfasst:
Anordnen von Sensormitteln (2) an dem Startknopf (1) in einem Bereich, den der Fahrer zum Anlassen berühren muss, zum Erkennen von substanzbezogenen Parametern, die einem Körperteil (3) des Fahrers zugeordnet sind, das für den Berührvorgang benutzt wird,
Vorsehen von Bestimmungsmitteln (4), um aus beliebigen der erkannten, dem Körperteil (3) zugeordneten, substanzbezogenen Parameter das Vorhandensein und die Konzentration von einer oder mehrerer Substanzen zu ermitteln, welche die Eignung des Fahrers zum Fahren negativ beeinflussen können,
Vorsehen von wählenden Anlassmitteln (5), die entweder das Anlassen des Motors des Fahrzeugs verhindern (6), falls ermittelt wird, dass eine oder mehrere Substanzen in einer Konzentration vorhanden ist oder sind, welche einen vorbestimmten Grenzwert übersteigt, oder andernfalls das Anlassen des Motors des Fahrzeugs zulassen (7).

## Revendications

1. Système destiné à permettre de façon sélective le démarrage du moteur d'un véhicule, le système comprenant un bouton de démarrage (1) disposé pour être pressé par un conducteur du véhicule pour démarrer un moteur de celui-ci,
**caractérisé en ce qu'**il comporte en outre :
- un moyen de détection (2), disposé au niveau du bouton de démarrage (1) en un endroit que le conducteur doit nécessairement presser pour le démarrage et destiné à détecter les paramètres se rapportant à une substance associés à une partie du corps (3) du conducteur utilisée pour l'action de pressage ;
- un moyen de détermination (4), destiné à déterminer, à partir de la détection de paramètres se rapportant à une substance et associés à cette partie du corps (3), la présence et la concentration d'une ou de plusieurs substances spécifiques capables d'affecter négativement la capacité de conduite du conducteur ;
- un moyen de démarrage sélectif (5), destiné soit à interdire (6) le démarrage du moteur du véhicule, lorsqu'il est déterminé que la présence d'une ou de plusieurs substances manifeste une concentration qui dépasse une valeur de seuil prédéterminée, ou sinon à permettre (7) le démarrage du moteur du véhicule.

2. Système selon la revendication 1, **caractérisé en ce que :**
le système est conçu pour détecter des paramètres se rapportant à l'alcool.

3. Système selon la revendication 2, **caractérisé en ce que :**
le système est conçu de façon à déterminer, à partir de paramètres se rapportant à l'alcool détectés sur la partie du corps (3) du conducteur utilisée dans l'action de pressage un taux d'alcool dans le sang du conducteur, et à comparer le taux déterminé d'alcool dans le sang avec la valeur de seuil prédéterminée.

4. Système selon la revendication 1, **caractérisé en ce que :**
le système est conçu pour détecter des paramètres se rapportant à une drogue.

5. Système selon la revendication 4, **caractérisé en ce que :**
le système est conçu pour détecter des paramètres se rapportant à une drogue à partir de la détection d'une ou plusieurs substances chimiques présentes sur la partie du corps (3) du conducteur utilisée dans l'action de pressage.

6. Système selon la revendication 5, **caractérisé en ce que :**
le système est conçu de façon à déterminer, à partir de paramètres se rapportant à une drogue détectés sur la partie du corps (3) du conducteur utilisée dans l'action de pressage, un taux de drogue dans le sang du conducteur, et à comparer le taux déterminé de drogue dans le sang avec la valeur de seuil prédéterminée.

7. Système selon l'une quelconque des revendications 1 à 6, **caractérisé en ce que :**
le système est conçu pour détecter des paramètres se rapportant à la somnolence.

8. Système selon la revendication 7, **caractérisé en ce que :**
le système est conçu pour détecter des paramètres se rapportant à la somnolence à partir de la détection d'une ou plusieurs hormones présentes sur la partie du corps (3) du conducteur utilisée dans l'action de pressage.

9. Véhicule automobile **caractérisé en ce que :**
il comporte un système selon l'une quelconque des revendications précédentes.

10. Procédé destiné à permettre de façon sélective le démarrage d'un moteur de véhicule, comprenant un bouton de démarrage (1) disposé de façon à être pressé par un conducteur du véhicule pour démarrer un moteur de celui-ci, **caractérisé en ce qu'**il comporte les étapes de :
- disposition d'un moyen de détection (2) au niveau du bouton de démarrage (1) en un endroit que le conducteur doit nécessairement presser pour le démarrage, et destiné à détecter les paramètres se rapportant à une substance associés à une partie du corps (3) du conducteur utilisée pour l'action de pressage ;
- installation d'un moyen de détermination (4), destiné à déterminer, à partir de la détection de paramètres se rapportant à une substance et associés à cette partie du corps (3), la présence et la concentration d'une ou de plusieurs substances spécifiques capables d'affecter négativement la capacité de conduite du conducteur ;
- installation d'un moyen de démarrage sélectif (5), destiné soit à interdire (6) le démarrage du moteur du véhicule, s'il a été déterminé que la présence d'une ou de plusieurs substances manifeste une concentration qui dépasse une valeur de seuil prédéterminée, ou sinon à permettre (7) le démarrage du moteur du véhicule.
